(19) **Europäisches Patentamt / European Patent Office / Office européen des brevets**

(11) **EP 3 675 716 B1**

(12) **FASCICULE DE BREVET EUROPEEN**

(45) Date de publication et mention de la délivrance du brevet:
**30.04.2025 Bulletin 2025/18**

(21) Numéro de dépôt: **18773532.9**

(22) Date de dépôt: **28.08.2018**

(51) Classification Internationale des Brevets (IPC):
**A61B 5/02** (2006.01)    **G16H 50/50** (2018.01)

(52) Classification Coopérative des Brevets (CPC):
**A61B 5/02028; A61B 5/725; G16H 50/50;**
A61B 5/4848

(86) Numéro de dépôt international:
**PCT/FR2018/052111**

(87) Numéro de publication internationale:
**WO 2019/043328 (07.03.2019 Gazette 2019/10)**

(54) **DISPOSITIF CARDIAQUE**

**KARDIALE VORRICHTUNG**

**CARDIAC DEVICE**

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **30.08.2017 FR 1758006**

(43) Date de publication de la demande:
**08.07.2020 Bulletin 2020/28**

(73) Titulaires:
- **INRIA - Institut National de Recherche en Informatique et en Automatique**
  **78150 Le Chesnay (FR)**
- **Assistance Publique Hôpitaux de Paris**
  **75004 Paris (FR)**

(72) Inventeurs:
- **CHABINIOK, Radomir**
  **75014 Paris (FR)**
- **CHAPELLE, Dominique**
  **75001 Paris (FR)**
- **LE GALL, Arthur**
  **75013 Paris (FR)**
- **MOIREAU, Philippe**
  **91190 Gif sur Yvette (FR)**

- **VALLEE, Fabrice**
  **93260 Les Lilas (FR)**

(74) Mandataire: **Vidon Brevets & Stratégie**
  **16B, rue de Jouanet**
  **BP 90333**
  **35703 Rennes Cedex 7 (FR)**

(56) Documents cités:
EP-A2- 3 043 276        CN-A- 105 852 841
US-A1- 2014 275 886     US-A1- 2017 235 915

- **M. CARUEL ET AL: "Dimensional reductions of a cardiac model for effective validation and calibration", BIOMECHANICS AND MODELING IN MECHANOBIOLOGY, vol. 13, no. 4, 8 December 2013 (2013-12-08), Berlin/Heidelberg, pages 897 - 914, XP055320070, ISSN: 1617-7959, DOI: 10.1007/s10237-013-0544-6**
- **CHIN-EN KUO ET AL: "Estimation and prediction of propafenone on the termination of atrial fibrillation by state-space models", COMPUTER SYMPOSIUM (ICS), 2010 INTERNATIONAL, IEEE, PISCATAWAY, NJ, USA, 16 December 2010 (2010-12-16), pages 841 - 845, XP031847660, ISBN: 978-1-4244-7639-8**

**Description**

L'invention concerne le domaine de la surveillance cardiaque en anesthésie-réanimation. L'art antérieur pertinent est décrit par exemple dans EP 3 043 276 A2, US 2017/0235915 A1, US 2014/0275886 A1, CN 105 852 841 A.

L'instabilité hémodynamique au bloc opératoire est fréquente et ses causes sont variées (chirurgie, insuffisance cardiaque, vasodilatation...). Une compréhension physiopathologique est nécessaire pour pouvoir anticiper, identifier, et traiter ces évènements et/ou leurs conséquences. Ainsi, il est recommandé de surveiller des indicateurs d'état et de fonction cardiovasculaire, comme la pression artérielle en continu et le débit cardiaque par doppler transoesophagien, des patients à risques d'évènements per-opératoires. Cependant, dans les cas les plus complexes, les analyses des mesures de la pression artérielle et des vélocités sanguines aortiques, ne rendant que partiellement comptent des interactions entre le cœur et les vaisseaux, peuvent être insuffisantes pour gérer au mieux la stratégie de réanimation cardiovasculaire au bloc opératoire.

La modélisation numérique permet par ailleurs de simuler le comportement du système cardiovasculaire, et d'extraire de la simulation des indicateurs normalement inaccessibles par surveillance non-invasive et/ou continue, comme la courbe pression/volume ventriculaire. Cependant, la qualité et la précision des indicateurs issus de ces simulations ne peuvent être obtenues qu'en exploitant conjointement des données suffisamment riches pour permettre d'adapter les paramètres du modèle, par des méthodes dites d'assimilation de données, afin d'approcher l'état cardiovasculaire du patient considéré. L'invention vient améliorer la situation. A cet effet, l'invention propose un dispositif cardiaque selon la revendication 1.

Ce dispositif permet d'améliorer la surveillance cardiovasculaire réalisée en anesthésie-réanimation, en utilisant des simulations en temps réel d'un modèle cardiovasculaire numérique, avec exploitation conjointe des données hémodynamiques de surveillance pour adapter le modèle en continu, et ainsi en extraire des indicateurs inaccessibles dans les données seules (par exemple des courbes pression/volume ventriculaires, la résistance vasculaire ou des contraintes myocardiques). En option, le modèle pourra comporter des entrées pharmacologiques permettant de prédire les effets des médicaments utilisés en anesthésie-réanimation. Ainsi, la simulation permettra également la mise en œuvre de boucles pro-actives d'administration des médicaments, conduisant à l'automatisation de leur administration.

L'invention présente en particulier les caractéristiques suivantes :

- le calculateur est agencé pour calculer un indicateur d'activité cardiaque, en appliquant un modèle cardiovasculaire pour calculer une valeur de pression artérielle et une valeur de débit cardiaque théoriques, et en appliquant au moins une fonction de correction basée sur la différence entre la valeur de pression artérielle et une valeur de débit cardiaque théoriques et des données hémodynamiques reçues dans la mémoire,
- le calculateur est agencé pour appliquer au moins un filtre de Kalman ou une combinaison d'un filtre de Kalman avec un observateur de Luenberger dans ladite au moins une fonction de correction, et
- optionnellement, le calculateur est en outre agencé pour appliquer le modèle de la circulation sanguine artérielle et veineuse avec un modèle pharmacologique.

L'invention concerne également un procédé de suivi cardiaque selon la revendication 3.

D'autres caractéristiques et avantages de l'invention apparaîtront mieux à la lecture de la description qui suit, tirée d'exemples donnés à titre illustratif et non limitatif, tirés des dessins sur lesquels :

- la figure 1 représente un schéma général d'un dispositif pour la surveillance cardiovasculaire en temps réel,
- la figure 2 représente un exemple de mise en œuvre d'une fonction exécutée par le dispositif de la figure 1,
- la figure 3 représente un exemple de mise en œuvre d'une opération de la figure 2, et
- les figures 4 à 8 représentent des exemples de signaux cliniques obtenus et exploités par le dispositif de la figure 1.

Les dessins et la description ci-après contiennent, pour l'essentiel, des éléments de caractère certain. Ils pourront donc servir à mieux faire comprendre la présente invention.

En outre, la description détaillée est augmentée de l'annexe A, qui donne la formulation de certaines formules mathématiques mises en œuvre dans le cadre de l'invention. Cette Annexe est mise à part dans un but de clarification, et pour faciliter les renvois. Elle est partie intégrante de la description, et sert à mieux faire comprendre la présente invention.

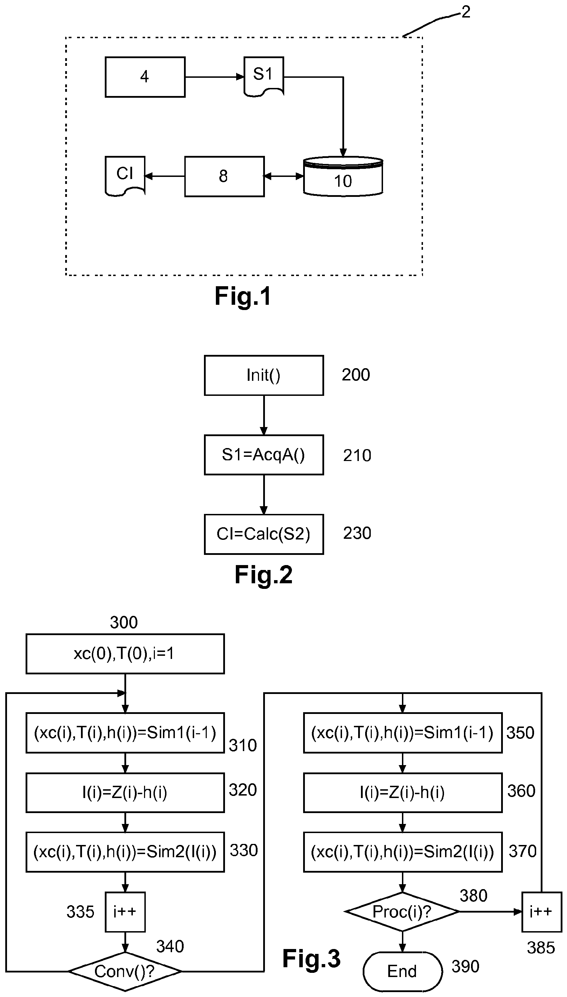

La figure 1 représente un schéma général d'un dispositif 2 de surveillance cardiovasculaire en temps réel. Le dispositif 2 comprend un système d'acquisition de mesures hémodynamiques 4, un calculateur 8 et une mémoire 10.

Dans l'exemple décrit ici, le dispositif 2 est un dispositif propre à être utilisé dans une salle d'opération. Plus particulièrement, il pourra être intégré à un appareil de surveillance anesthésique existant, afin d'économiser de l'espace. En variante, le dispositif 2 pourrait être réalisé séparément, et recevoir les données de surveillance anesthésique nécessaire à son fonctionnement. Toujours en variante, les éléments du dispositif 2 pourraient être réalisés séparément.

Le calculateur 8 est un élément accédant directement ou indirectement à la mémoire 10. Il peut être réalisé sous la forme d'un code informatique approprié exécuté sur un ou plusieurs processeurs. Par processeurs, il doit être compris tout processeur adapté. Un tel processeur peut être réalisé de toute manière connue, sous la forme d'un microprocesseur pour ordinateur personnel, d'une puce dédiée de type FPGA ou SoC (« system on chip » en anglais), d'une ressource de calcul sur une grille, d'un microcontrôleur, ou de toute autre forme propre à fournir la puissance de calcul nécessaire à la réalisation décrite plus bas. Un ou plusieurs de ces éléments peuvent également être réalisés sous la forme de circuits électroniques spécialisés tel un ASIC. Une combinaison de processeur et de circuits électroniques peut également être envisagée.

La mémoire 10 peut être tout type de stockage de données propre à recevoir des données numériques : disque dur, disque dur à mémoire flash (SSD en anglais), mémoire flash sous toute forme, mémoire vive, disque magnétique, stockage distribué localement ou dans le cloud, etc. Les données calculées par le dispositif peuvent être stockées sur tout type de mémoire similaire à la mémoire 10, ou sur celle-ci. Ces données peuvent être effacées après que le dispositif a effectué ses tâches, ou conservées. Dans l'exemple décrit ici, le système d'acquisition de mesures hémodynamiques 4 est un moniteur de type Philips Intellivue MP60 permettant de connecter différentes sources électroniques, mesurant elles-mêmes des indicateurs hémodynamiques (comme la pression artérielle, ou le débit cardiaque). Le système d'acquisition de mesures hémodynamiques 4 mesure en temps réel la pression artérielle et le débit aortique.

Le modèle cardiovasculaire comprend :

- un modèle cardiaque reliant des paramètres de modélisation du cœur (module d'élasticité, contractilité, tension active maximale des fibres musculaires, etc.) et des variables représentatives de l'état du cœur, notamment décrivant ses déformations, et
- un modèle de la circulation sanguine artérielle et veineuse reliant également des paramètres (élastances artérielles, résistances périphériques, etc.), et des variables d'état (notamment pressions artérielles et veineuses).

Dans le cas décrit ici, les grandeurs cardiaques qu'un clinicien souhaitera surveiller comprennent la variation du volume du ventricule gauche (relié à la variation du rayon de la cavité, ci-après notée y), la déformation des fibres musculaires (ci-après ec), la raideur active cardiaque (ci-après kc), la contrainte active cardiaque (ci-après tc), la pression aortique (ci-après Par) et la pression artérielle distale (ci-après Pd). Ces grandeurs caractéristiques de l'état de fonctionnement cardiaque seront dans la suite rassemblées dans un vecteur xc. D'autres grandeurs d'intérêt concernent certains paramètres de modélisation susceptibles d'évoluer au fil du temps en fonction de l'état du patient, et ces paramètres comprennent notamment la contractilité (ci-après s0), la raideur active (ci-après k0) et la résistance périphérique (ci-après Rd). Ces paramètres sont rassemblés dans un vecteur noté T. Enfin, des indicateurs variés peuvent être tirés du vecteur xc et des paramètres de modélisation T pour permettre de suivre l'évolution du système cardiovasculaire. Ces indicateurs, comprenant notamment le débit cardiaque (ci-après Qc), seront dans la suite désignés par un vecteur CI.

Le vecteur xc et le vecteur T sont reliés par les équations (10) à (70) de l'Annexe A, dans laquelle les équations (10), (20), (30), (40), (50), (60) et (70) désignent les relations indépendantes, et les équations (11) à (18) définissent des membres de l'équation (10). La grandeur $\underline{u}$ désigne une fonction du temps fixée (fonction d'activation), et n0 une fonction de la déformation ec modulant la tension active (effet Starling). L'article de M. Caruel et al, « Dimensional reductions of a cardiac model for effective validation and calibration », Biomech Model Mechanobiol, 2013 décrit ces équations en détail.

Le système formé par les équations (10) à (70) peut être résumé sous la forme de l'équation (80).

Une fois ce modèle cardiaque posé, un modèle de couplage artério-veineux peut être utilisé. Ce modèle permet d'établir une valeur de pression atriale Pat (également appelée précharge). En effet, la pression atriale ne peut pas être connue par prises en continu, et est un indicateur extrêmement utile de l'état cardiaque d'un patient.

Le couplage artério-veineux s'exprime en établissant d'une part l'équilibre entre le débit du compartiment artériel et du compartiment veineux, et en utilisant la définition du débit du compartiment veineux comme un lissage du débit cardiaque.

Il s'en suit l'équation (90), dans laquelle le membre de gauche représente le flux sanguin traversant les capillaires, fonction de la différence de pression entre le compartiment artériel et le compartiment veineux et utilisant une relation de conservation du volume sanguin, et le membre de droite représente la définition du débit du compartiment veineux (Qsv) comme un lissage du débit cardiaque (Qc), tel que vu par les capillaires.

Dans le membre de gauche, la valeur Veff représente le volume sanguin effectif participant aux échanges artério-veineux, tandis que la valeur Csv représente la compliance du système veineux, ou capacité de stockage côté veineux. Dans le membre de droite, la formule exponentielle sert à modéliser un effet mémoire afin de lisser le débit dans les veines lorsque le cœur n'expulse pas de sang, et l'expression au dénominateur représente une constante de normalisation. L'article de D. Chapelle et al, « Patient-specific biomechanical modeling of cardiac amyloidosis - A case study », Proc. of FIMH 2015, LNCS Vol. 9126, pp. 295-303, Springer 2015 décrit ces équations en détail.

L'équation (90) peut donc être utilisée pour déterminer la pression dans le système veineux (Psv), et en tirer la pression atriale (Pat) conformément à l'équation (100) de l'Annexe A. Dans cette équation, une fonction est ajoutée à la pression du système veineux afin de tenir compte du surcroît de pression induit par la contraction de l'oreillette au début du cycle cardiaque. Cette fonction est typiquement une rampe montante, suivie d'un plateau et d'une rampe descendante, qui se répète avec chaque cycle cardiaque.

Dans une situation idéale, il serait donc possible d'utiliser ces équations pour tout déterminer instantanément. Pour cela, il convient de partir des mesures, définies par la mesure théorique z(t) dans l'équation (110) de l'Annexe A. Mais, si l'on peut comparer les simulations du modèle aux mesures, cette chaîne d'opérations n'est pas inversible on ne peut pas ainsi directement déterminer l'état et les paramètres du modèle pour le faire correspondre à l'état courant du patient.

Afin de pallier ce problème, lié au fait que l'opérateur Ac n'est pas nécessairement inversible, la Demanderesse a conçu une fonction Calc() qui vient compléter le modèle décrit plus haut, et a discrétisé ce dernier.

Pour ajuster en continu le modèle au patient, la Demanderesse a introduit avec l'équation (120) un vecteur d'innovation qui mesure la différence entre la mesure débruitée Z(t) et la mesure théorique z(t). Le vecteur d'innovation est ensuite réintroduit dans des fonctions de corrections basées sur un filtre de Kalman, ou sur une combinaison d'un filtre de Kalman avec un observateur de Luenberger comme exposé dans l'article de Moireau et al. « Joint state and parameter estimation for distributed mechanical systems », Comput. Methods Appl. Mech. Engrg. 197 (2008) 659-677. Ces fonctions de correction ont l'avantage d'assurer une convergence dans une large gamme de situations, en quelques boucles de fonctionnement.

En conséquence la Demanderesse a introduit l'équation (180) pour modifier l'équation (80) afin de tenir compte de ces corrections, et introduit l'équation (185) pour tenir compte de leurs effets sur le vecteur T.

Le calculateur 8 peut également recevoir des signaux mesurés par d'autres capteurs, notamment des mesures de pression non-invasive localisée par exemple au doigt ou à la carotide commune, des données issues du respirateur (pression des voies aériennes, débit d'insufflation, volume courant etc...), des données électroencéphalographiques et électrocardiographiques, oxymétrie cérébral par spectroscopie en proche-infrarouge, la saturation pulsée en oxygène ou mesures cutanées de pression en oxygène ou $CO_2$, ou des mesures invasives de pression et de flux obtenues par cathétérisme direct, mais aussi la pression intracrânienne. Ces mesures complémentaires sont alors combinées aux données cardiaques déterminées par le calculateur 8 pour en tirer des indicateurs cardiaques permettant le suivi de maladies cardiaques spécifiques.

La figure 2 représente un exemple d'une fonction mise en œuvre par le dispositif 2. Dans une opération 200, le dispositif 2 exécute une fonction Init(). La fonction Init() initialise le dispositif 2, notamment en sélectionnant des paramètres spécifiques au patient relatifs au modèle cardiovasculaire. Cette fonction peut réaliser une opération de calibration à chaque allumage du dispositif 2, ou réaliser cette calibration de manière périodique ou une fois pour toutes.

Ensuite, dans une opération de 210, l'acquisition de signaux hémodynamiques est réalisée. Dans l'exemple décrit ici, il s'agit de variables hémodynamiques artérielles, à savoir la pression artérielle et le flux aortique.

Enfin, dans une opération de 230, le calculateur 8 exécute une fonction Calc() qui applique le modèle cardiovasculaire aux signaux pour en tirer les données cardiaques CI.

La figure 3 représente un exemple de mise en œuvre de la fonction Calc().

La fonction Calc() comprend deux boucles au fonctionnement identique, dont la première sert à l'initialisation et la deuxième à l'exploitation.

Dans une opération 300, la fonction Calc() débute avec des valeurs d'initialisation des vecteurs xc et T, ainsi qu'avec un indice temporel i.

Ensuite, dans une opération 310, une fonction Sim1() est appliquée avec comme argument l'indice i-1. La fonction Sim1() applique de manière séquentielle une version discrétisée des équations (80) à (110), afin de déterminer quelle serait la valeur de z(t) pour l'instant correspondant à l'instant i, telle que calculée selon le modèle théorique. Cette valeur est désignée par h(i).

Les équations (280) et (310) représentent une possible version discrétisée, par une méthode dite explicite, des équations (80) et (110) appliquées par la fonction Sim1(). Ensuite, dans une opération 320, le calculateur 8 calcule le vecteur d'innovation en appliquant l'équation (120), et la simulation est répétée dans une opération 330 avec une fonction Sim2().

La fonction Sim2() reçoit le vecteur d'innovation I(i) pour appliquer les équations (180) et (185), sous une forme discrétisée de la même manière que les équations (280) et (310), comme cela apparaît avec les équations (380) à (410). Dans la pratique, la fonction Sim2() applique une correction tirée du vecteur d'innovation aux calculs déjà réalisés avec la fonction Sim1(). En variante, la fonction Sim2() pourrait reprendre intégralement les calculs.

Ensuite, dans une opération 335, l'indice i est incrémenté, et, une fonction Conv() est exécutée dans une opération 340 pour comparer la différence entre la valeur h(i) issue de l'opération 330 et la mesure Z(i) issue des signaux hémodynamiques utilisés à l'opération 320.

Lorsque cette différence est supérieure à un seuil choisi, alors il est considéré que les fonctions de correction ne suffisent pas encore, et la fonction Calc() reprend avec l'opération 310.

Comme mentionné précédemment, la convergence par les fonctions de corrections est atteinte assez rapidement, par exemple à partir des mesures hémodynamiques associées à un unique battement cardiaque.

Lorsque la différence est inférieure au seuil choisi, alors il est considéré que les valeurs déterminées sont utiles, et la deuxième boucle commence.

Dans la deuxième boucle, des opérations 350 à 370 identiques aux opérations 310 à 330 sont exécutées afin de déterminer les valeurs courantes du vecteur xt(i) et T(i). Ensuite, dans une opération 380, ces mesures sont affichées et/ou font l'objet d'un traitement relatif au suivi de la maladie avec l'exécution d'une fonction Proc(). La fonction Proc() détermine également s'il reste des données à traiter. Si c'est le cas, alors l'indice i est incrémenté dans une opération 385, et la deuxième boucle reprend avec l'opération 350. Sinon, la fonction Calc() se termine dans une opération 390.

Sur la base des calculs de la fonction Calc() un ensemble de fonctionnalités peuvent être mises en œuvre, comprenant l'émission d'un signal d'avertissement, l'affichage des vecteurs xc et T calculés, le calcul d'indicateurs cardiaques CI à partir des vecteurs xc et T, etc.

De plus, le modèle décrit plus haut peut être complété afin d'intégrer l'introduction d'éléments pharmacologiques dans le système veineux. Les effets de ces éléments peuvent être également être pris en compte, afin de permettre à un médecin de prévoir l'évolution du patient faisant l'objet de la surveillance en fonction de l'élément pharmacologique introduit et de sa quantité.

Dans l'exemple décrit ici, la Demanderesse présente l'exemple de trois éléments :

- le propofol, à effet anesthésique,
- la noradrénaline, à effet vasopresseur, et
- l'infusion de fluides, qui ajoute directement du volume liquide dans la circulation.

Dans le troisième cas, l'effet peut directement être déterminé dans ce qui a été décrit précédemment. En effet, l'ajout de volume liquide dans la circulation vient modifier la valeur Veff, et donc la discrétisation des équations (90) et (100).

Dans le premier et le deuxième cas, l'effet de l'élément pharmacologique sur un paramètre K considéré est modélisé selon l'équation (500), où $\alpha(t)$ est une loi d'évolution de l'élément selon l'équation (510) de l'Annexe A, et K0 une valeur de base du paramètre K considéré. La loi $\alpha(t)$ pourra varier en fonction de la valeur de K0, afin de tenir compte d'un effet de saturation.

Dans l'équation (510), X représente la concentration de l'élément pharmacologique introduit, $\alpha_\infty(X)$ l'augmentation de K en valeur relative obtenue par une infusion prolongée de l'élément pharmacologique en concentration X, et Tx est une constante de temps qui définit la vitesse d'effet de l'élément pharmacologique en concentration X.

Dans le cas du propofol (respectivement de la noradrénaline), les paramètres cardiaques qui seront influencés selon les équations (500) et (510) seront la contractilité cardiaque s0, la résistance distale Rd et la capacité distale Cd. Le propofol (respectivement la noradrénaline) a pour effet de baisser (respectivement augmenter) la résistance distale Rd, et d'augmenter (respectivement baisser) la capacité distale Cd. De plus, la noradrénaline a pour effet d'augmenter la contractilité cardiaque s0. Il y aura donc un jeu d'équations par paramètre influencé.

Ici encore, ces équations viendront modifier la discrétisation des équations (90) et (100), ainsi que celle des équations (30) à (70), et l'équation (510) sera discrétisée selon l'équation (520) de l'Annexe A.

Les lois $\alpha(t)$, $\alpha_\infty(X)$ et les constantes Tx pourront être déterminées au préalable, par moyenne sur un groupe d'individus, et optionnellement faire l'objet d'une adaptation par individu le cas échéant.

La fonction de calcul peut mettre en œuvre d'autres modèles cardiovasculaires que celui donné ici à titre d'exemple, et recevoir et prétraiter des signaux mesurés par d'autres capteurs, et notamment ceux permettant de mesurer des indicateurs renseignant sur l'adéquation entre la consommation et les apports d'oxygène au niveau d'organes tels que le cerveau, le cœur, le rein et les tissus musculo-cutanés.

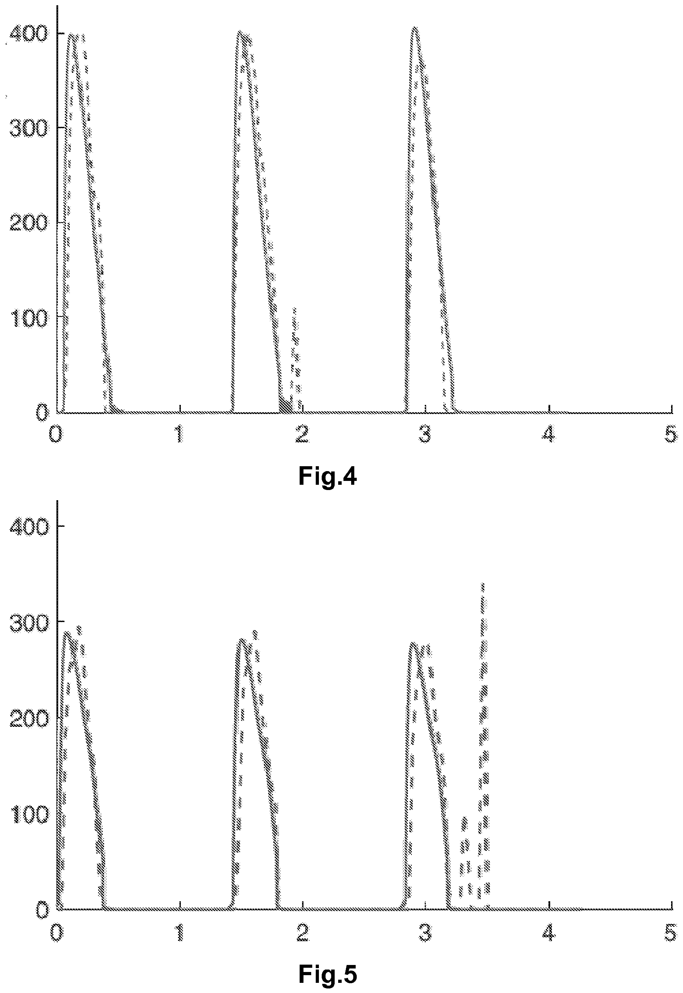

Les figures 4 et 5 représentent en abscisse le temps en s, et en ordonnée le flux aortique en ml/s. Sur ces deux figures, le tracé en pointillés représente les valeurs mesurées, et le tracé en trait plein représente les valeurs simulées par le dispositif de la figure 1. La figure 4 représente un cas sans infusion de noradrénaline, tandis que la figure 5 représente un cas avec infusion de noradrénaline.

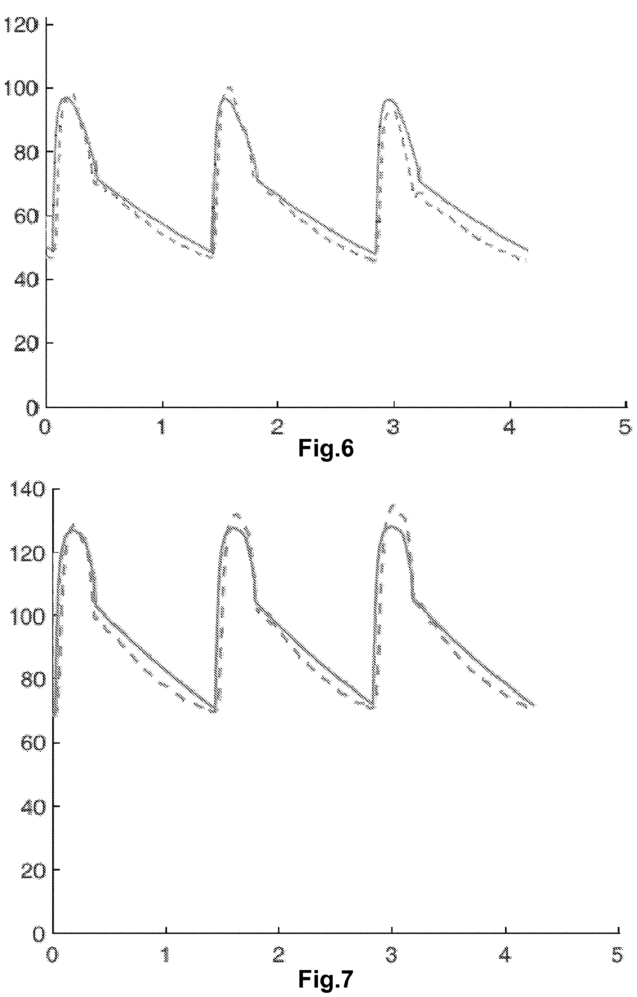

Les figures 6 et 7 représentent en abscisse le temps en s, et en ordonnée la pression aortique en mmHg. Sur ces deux figures, le tracé en pointillés représente les valeurs mesurées, et le tracé en trait plein représente les valeurs simulées par le dispositif de la figure 1. La figure 6 représente un cas sans infusion de noradrénaline, tandis que la figure 7 représente un cas avec infusion de noradrénaline.

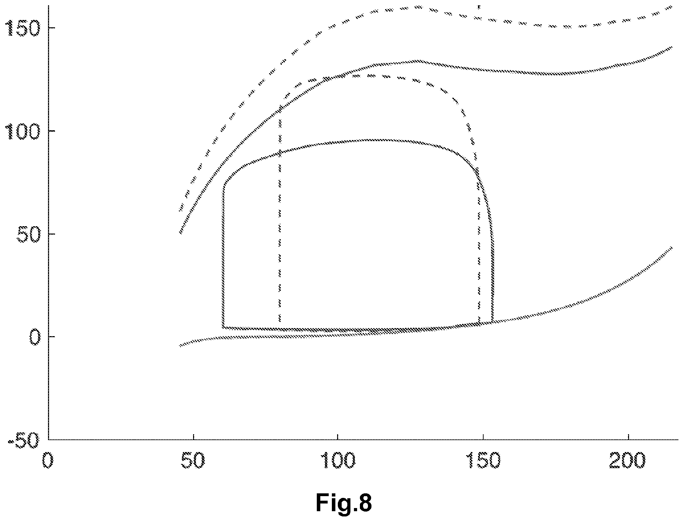

Enfin, la figure 8 représente des boucles pression ventriculaire (en ordonnée, en mmHg)-volume ventriculaire (en abscisse, en ml) calculées par le modèle calibré à partir des mesures et relations pression -volume diastolique / systolique. Sur cette figure, les tracés en traits pleins représentent les boucles pendant un état de repos, et les tracés en pointillés représentent les boucles pendant une administration de noradrénaline.

Les tracés fermés représentent la boucle pression-volume, les tracés dans la partie supérieure représentent la relation pression télésystolique-volume, et les tracés dans la partie inférieure représentent la relation pression télé-

diastolique-volume.

ANNEXE A

$$\rho d_0 \ddot{y} + \frac{d_0}{R_0}\left(1 + \frac{y}{R_0}\right)\Sigma_{sph} = P_v\left(1 + \frac{y}{R_0}\right)^2 \tag{10}$$

$$\Sigma_{sph} = \sigma_{1D} + 4(1 - C^{-3})\left(\frac{\partial W_e}{\partial J_1} + C\frac{\partial W_e}{\partial J_2}\right) + 2\frac{\partial W_e}{\partial J_4} + 2\eta \dot{C}(1 - 2C^{-6}) \tag{11}$$

$$\sigma_{1D} = E_S\frac{e_{1D} - e_c}{(1 + 2e_c)^2} \tag{12}$$

$$C = \left(1 + \frac{y}{R_0}\right)^2 \tag{13}$$

$$e_{1D} = \frac{C - 1}{2} \tag{14}$$

$$f_{va}(P_v, P_{ar}, P_{at}) = -4\pi R_0{}^2\left(1 + \frac{y}{R_0}\right)^2 \dot{y} \tag{15}$$

$$J_1 = 2C + C^{-2} \tag{16}$$

$$J_4 = C \tag{17}$$

$$W_e(J_1, J_4) = k_1 e^{k_2(J_1 - 3)^2} + k_3 e^{k_4(J_4 - 1)^2} \tag{18}$$

$$(t_c + \mu\dot{e}_c) = E_S\frac{(e_{1D} - e_c)(1 + 2e_{1D})}{(1 + 2e_c)^3} \tag{20}$$

$$\dot{k}_c = -(|\bar{u}| + \alpha|\dot{e}_c|)k_c + n_0 k_0 |\bar{u}|_+ \tag{30}$$

$$\dot{t}_c = -(|\bar{u}| + \alpha|\dot{e}_c|)t_c + n_0 s_0 |\bar{u}|_+ + k_c\dot{e}_c \tag{40}$$

$$C_p\dot{P}_{ar} + \frac{(P_{ar} - P_d)}{R_p} = Q_c \tag{50}$$

$$Q_c = \begin{cases} f_{va}(P_v, P_{ar}, P_{at}) \ si \ f_{va}(P_v, P_{ar}, P_{at}) > 0 \\ 0 \ sinon \end{cases} \tag{60}$$

$$C_d\dot{P}_d + \frac{(P_d - P_{ar})}{R_p} = \frac{(P_{sv} - P_d)}{R_d} \tag{70}$$

$$\dot{x}_c = A_c(x_c, T, t) \tag{80}$$

$$\frac{V_{eff}}{R_d C_d} - \left(1 + \frac{C_{sv}}{C_d}\right)\frac{P_{sv}(t)}{R_d} = \frac{\int_{t-\Delta T}^{t} e^{-\frac{t-s}{T0}} Q_c(s)ds}{\int_{t-\Delta T}^{t} e^{-\frac{t-s}{T0}} ds} \tag{90}$$

$$P_{at}(t) = P_{sv}(t) + \Delta P_{at}(t) \tag{100}$$

$$z(t) = \begin{bmatrix} P_{ar} \\ Q_c \end{bmatrix} \tag{110}$$

$$I(t) = Z(t) - z(t) \tag{120}$$

$$\dot{x}_c = A_c(x_c, T, t) + K_c(I(t), t) \tag{180}$$

$$\dot{T} = K_T(I(t), t) \tag{185}$$

$$x_c(t_{i+1}) = A_c(x_c(t_i), T(t_i), t_i)(t_{i+1} - t_i) + x_c(t_i) \tag{280}$$

$$z(t_{i+1}) = \begin{bmatrix} \frac{P_{ar}(t_{i+1}) - P_{ar}(t_i)}{(t_{i+1} - t_i)} \\ \frac{Q_c(t_{i+1}) + Q_c(t_i)}{2} \end{bmatrix} \tag{310}$$

$$x_c(t_{i+1}) = (A_c(x_c(t_i), T(t_i), t_i) + K_c(I(t_{i+1}), t_{i+1}))(t_{i+1} - t_i) + x_c(t_i) \tag{380}$$

$$T(t_{i+1}) = K_T(I(t_{i+1}), t_{i+1})(t_{i+1} - t_i) + T(t_i) \tag{385}$$

$$z(t_{i+1}) = \begin{bmatrix} \frac{P_{ar}(t_{i+1}) - P_{ar}(t_i)}{(t_{i+1} - t_i)} \\ \frac{Q_c(t_{i+1}) + Q_c(t_i)}{2} \end{bmatrix} \tag{410}$$

$$K(t) = (1 + \alpha(t))K_0 \tag{500}$$

$$\dot{\alpha}(t) + \frac{\alpha(t)}{T_X} = \frac{\alpha_\infty(X)}{T_X} \tag{510}$$

$$\frac{\alpha(t_{i+1}) - \alpha(t_i)}{(t_{i+1} - t_i)} + \frac{\alpha(t_{i+1}) + \alpha(t_i)}{2T_X} = \frac{\alpha_\infty(X(\frac{t_{i+1} + t_i}{2}))}{T_X} \tag{520}$$

## Revendications

1. Dispositif cardiaque pour la surveillance cardiovasculaire en temps réel réalisée en anesthésie-réanimation **caractérisé en ce qu'**il comprend une mémoire (10) agencée pour recevoir des données hémodynamiques, et un calculateur (8) agencé pour appliquer un modèle cardiovasculaire comprenant un modèle cardiaque et un modèle de la circulation sanguine artérielle et veineuse à partir des données reçues dans la mémoire (10), et pour en tirer au moins un indicateur (CI) d'activité cardiaque **caractérisé en ce que** le calculateur (8) est agencé pour calculer l'au moins un indicateur d'activité cardiaque (CI) en appliquant le modèle cardiovasculaire pour calculer une valeur de pression artérielle et une valeur de débit cardiaque théoriques, et en appliquant au moins une fonction de correction basée sur la différence entre la valeur de pression artérielle et une valeur de débit cardiaque théoriques et des données hémodynamiques reçues dans la mémoire (10), le calculateur (8) appliquant au moins un filtre de Kalman ou une combinaison d'un filtre de Kalman avec un observateur de Luenberger dans ladite au moins une fonction de correction.

2. Dispositif selon la revendication 1, dans lequel le calculateur (8) est en outre agencé pour appliquer le modèle de la circulation sanguine artérielle et veineuse avec un modèle pharmacologique.

3. Procédé de suivi cardiaque pour la surveillance cardiovasculaire en temps réel réalisée en anesthésie-réanimation comprenant :

   - recevoir des données hémodynamiques,
   - appliquer un modèle cardiovasculaire comprenant un modèle cardiaque et un modèle de la circulation sanguine artérielle et veineuse aux données hémodynamiques, et en tirer au moins un indicateur (CI) d'activité cardiaque,

   **caractérisé en ce que** le calcul de l'au moins un indicateur d'activité cardiaque (CI) comprend l'application du modèle cardiovasculaire pour calculer une valeur de pression artérielle et une valeur de débit cardiaque théoriques, et l'application d'au moins une fonction de correction basée sur la différence entre la valeur de pression artérielle et une valeur de débit cardiaque théoriques et des données hémodynamiques reçues dans la mémoire (10), au moins un filtre de Kalman ou une combinaison d'un filtre de Kalman avec un observateur de Luenberger étant appliquée dans ladite au moins une fonction de correction.

**Patentansprüche**

1. Kardiale Vorrichtung zur kardiovaskulären Echtzeitüberwachung, die in der Anästhesie-Reanimation ausgeführt wird, **dadurch gekennzeichnet, dass** sie einen Speicher (10) umfasst, der dazu ausgestaltet ist, hämodynamische Daten zu empfangen, und einen Rechner (8), der dazu ausgestaltet ist, ein kardiovaskuläres Modell, das ein kardiales Modell und ein Modell der arteriellen und venösen Blutzirkulation umfasst, ausgehend von den in dem Speicher (10) empfangenen Daten anzuwenden und daraus mindestens einen Indikator für die kardiale Aktivität (CI) abzuleiten **dadurch gekennzeichnet, dass** der Rechner (8) dazu ausgestaltet ist, den mindestens einen Indikator für die kardiale Aktivität (CI) zu berechnen, indem er das kardiovaskuläre Modell anwendet, um einen theoretischen arteriellen Druckwert und einen theoretischen Herzzeitvolumenwert zu berechnen, und indem er mindestens eine Korrekturfunktion anwendet, die auf der Differenz zwischen dem theoretischen arteriellen Druckwert und einem theoretischen Herzzeitvolumenwert und in dem Speicher (10) empfangenen hämodynamischen Daten basiert, wobei der Rechner (8) mindestens ein Kalman-Filter oder eine Kombination eines Kalman-Filters mit einem Luenberger-Beobachter in der mindestens einen Korrekturfunktion anwendet.

2. Vorrichtung nach Anspruch 1, wobei der Rechner (8) ferner dazu ausgestaltet ist, das Modell der arteriellen und venösen Blutzirkulation mit einem pharmakologischen Modell anzuwenden.

3. Verfahren zum kardialen Monitoring zur kardiovaskulären Echtzeitüberwachung in der Anästhesie-Reanimation, umfassend:

   - Empfangen von hämodynamischen Daten,
   - Anwenden eines kardiovaskulären Modells, das ein kardiales Modell und ein Modell der arteriellen und venösen Blutzirkulation umfasst, auf die hämodynamischen Daten, und Ableiten mindestens eines Indikators für die kardiale Aktivität (CI) daraus,

   **dadurch gekennzeichnet, dass** das Berechnen des mindestens einen Indikators für die kardiale Aktivität (CI) das Anwenden des kardiovaskulären Modells umfasst, um einen theoretischen arteriellen Druckwert und einen theoretischen Herzzeitvolumenwert zu berechnen, und das Anwenden mindestens einer Korrekturfunktion, die auf der Differenz zwischen dem theoretischen arteriellen Druckwert und einem theoretischen Herzzeitvolumenwert und in dem Speicher (10) empfangenen hämodynamischen Daten basiert, wobei mindestens ein Kalman-Filter oder eine Kombination eines Kalman-Filters mit einem Luenberger-Beobachter in der mindestens einen Korrekturfunktion angewandt wird.

**Claims**

1. Cardiac device for real-time cardiovascular monitoring carried out in anaesthesia and intensive care **characterized in that** the device comprises a memory (10) arranged for receiving haemodynamic data, and a calculator (8) arranged for applying a cardiovascular model comprising a cardiac model and an arterial and venous blood circulation model based on the data received in the memory (10), and for deriving therefrom at least one cardiac activity indicator (CI), **characterized in that** the calculator (8) is arranged for computing the at least one cardiac activity indicator (CI), by applying a cardiovascular model to compute an arterial pressure value and a cardiac output value that are theoretical, and by applying at least one correction function based on the difference between the arterial pressure value and a cardiac output value that are theoretical and haemodynamic data received in the memory (10), the calculator (8) applying at least one Kalman filter or a combination of a Kalman filter with a Luenberger observer in said at least one correction function.

2. Device according to claim 1, wherein the calculator (8) is further arranged for applying the arterial and venous blood circulation model with a pharmacological model.

3. Cardiac monitoring method for real-time cardiovascular monitoring carried out in anaesthesia and intensive care comprising:

   - receiving haemodynamic data,
   - applying a cardiovascular model comprising a cardiac model and an arterial and venous blood circulation model to the haemodynamic data, and deriving therefrom at least one cardiac activity indicator (CI),

   **characterized in that** the computation of the at least one cardiac activity indicator (CI) comprises applying of a cardiovascular model to compute an arterial pressure value and a cardiac output value that are theoretical, and applying of at least one correction function based on the difference between the arterial pressure value and a cardiac output value that are theoretical and haemodynamic data received in the memory (10), at least one Kalman filter or a combination of a Kalman filter with a Luenberger observer being applied in said at least one correction function.

**Fig.1**

| | |
|---|---|
| Init() | 200 |
| S1=AcqA() | 210 |
| CI=Calc(S2) | 230 |

**Fig.2**

300
xc(0),T(0),i=1

(xc(i),T(i),h(i))=Sim1(i-1) | 310
I(i)=Z(i)-h(i) | 320
(xc(i),T(i),h(i))=Sim2(I(i)) | 330
335 | i++
340
Conv()?

(xc(i),T(i),h(i))=Sim1(i-1) | 350
I(i)=Z(i)-h(i) | 360
(xc(i),T(i),h(i))=Sim2(I(i)) | 370
Proc(i)? | 380
i++
385
End | 390

**Fig.3**

**Fig.4**

**Fig.5**

**Fig.6**

**Fig.7**

**Fig.8**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 3043276 A2
- US 20170235915 A1
- US 20140275886 A1
- CN 105852841 A

**Littérature non-brevet citée dans la description**

- **M. CARUEL et al.** Dimensional reductions of a cardiac model for effective validation and calibration. *Biomech Model Mechanobiol*, 2013
- Patient-specific biomechanical modeling of cardiac amyloidosis - A case study. **D. CHAPELLE et al.** Proc. of FIMH 2015, LNCS. Springer, 2015, vol. 9126, 295-303
- **MOIREAU et al.** Joint state and parameter estimation for distributed mechanical systems. *Comput. Methods Appl. Mech. Engrg.*, 2008, vol. 197, 659-677